Europäisches Patentamt

European Patent Office

Office européen des brevets

(19)

(11) Publication number: **0 172 671**
**A2**

(12) # EUROPEAN PATENT APPLICATION

(21) Application number: **85305297.5**

(22) Date of filing: **25.07.85**

(51) Int. Cl.⁴: **A 61 C 15/04**
**A 61 K 7/16**

(30) Priority: **26.07.84 US 634719**

(43) Date of publication of application:
**26.02.86 Bulletin 86/9**

(84) Designated Contracting States:
**AT CH GB IT LI SE**

(71) Applicant: **JOHNSON & JOHNSON Products Inc,**
**One Johnson & Johnson Plaza**
**New Brunswick, NJ 08933-7003(US)**

(72) Inventor: **Finkelstein, Paul**
**10 Springwood Drive**
**Princeton Junction New Jersey 08850(US)**

(72) Inventor: **Yost, Kevin Gardner**
**22 Glenwood Drive**
**Short Hills New Jersey 07078(US)**

(74) Representative: **Jones, Alan John et al,**
**CARPMAELS & RANSFORD 43 Bloomsbury Square**
**London, WC1A 2RA(GB)**

(54) **Dental floss.**

(57) A dental floss consisting of a single end yarn containing
from about 25 to 1000 filaments.

EP 0 172 671 A2

# DENTAL FLOSS

## BACKGROUND OF THE INVENTION

This invention relates to articles for cleaning the interproximal surfaces of the teeth and more particularly to dental floss.

It has been shown that tooth decay and dental disease can be attributed to bacterial action resulting from the formation of plaque about the teeth and/or the entrapment of food particles between the teeth. The removal of plaque and entrapped food particles reduces caries, reduces the tendency towards gingivitis, and reduces mouth odor as well as generally improving oral hygiene. Conventional brushing of the teeth has been found to be unsatisfactory to effect the removal of entrapped food particles from some crevices between the teeth and/or to effectively remove the bacterial plaque.

To supplement brushing of the teeth, various materials have been used to clean the interproximal spaces and surfaces of the teeth, for example dental floss and dental tape. Dental floss is generally comprised of multifilament yarns. Smaller yarns, also referred to as ends, are comprised of many filaments extruded as a bundle and commonly held together by twisting. A number of these ends, each an individual entity, are then combined and twisted together to form a larger yarn which is then coated with a bonding material to form the floss commonly available commercially. While satisfactory for many users, such floss does present problems for some who find it difficult to insert the floss into tight spaces between the teeth. This is due in part to the inability

-1152

of the various filaments to easily slide over one another as the floss is forced between contacting teeth. The intrayarn sliding of the filaments is inhibited by virtue of being twisted into one of a number of individual ends.

## SUMMARY OF THE INVENTION

It is an object of this invention to provide an improved dental floss.

It is a further object of this invention to provide a dental floss allowing easier insertion into tight spaces between the teeth.

Other objects of the present invention will be set forth in, or apparent from, the following detailed description of the invention.

The foregoing objects and other features and advantages of the present invention are achieved by a dental floss consisting of a single end yarn containing from about 25 to 1,000 filaments and having a twist of from about 0 to 6 turns per inch.

## DESCRIPTION OF PREFERRED EMBODIMENTS

A preferred embodiment of the present invention comprises a single end yarn dental floss formed of a number of individual filaments of a substrate material suitable for use as a dental floss. The suitable substrate materials are those which are capable of being extruded by standard extrusion technology and include high and normal tenacity nylon such as nylon 6 and 6,6, rayon, acetate polymers, polyester, polyethylene, polypropylene and any other extrudable plastic. From about 25 to 1,000 filaments,

J&J-1152

preferably about 55 to 450 filaments are formed together into a single end yarn of a sufficiently small diameter to permit insertion into the interproximal areas between closely contacting teeth. If desired, the filaments can be colored utilizing any suitable dye such as FD&C Blue No. 1, FD&C Yellow No. 10, FD&C Green No. 3, FD&C Red No. 40 or mixtures of these or other suitable dyes. The filaments can also be colored by placing a suitable pigment, such as naphthol red, phthalocyanine green, diarylide yellow or the like in the polymer melt prior to extrusion of the filaments.

It is common practice to twist the individual filaments to form the single end yarn in order to give the floss additional integrity, that is additional strength to prevent shredding and filament separation. The twist incorporated into the single end yarn should be enough to aid handling and keep the filaments together during use, but should not be so great that the floss becomes stiff and inflexible. The twist of the yarn can be from about 0 to 6 turns per inch, with a preferred twist of from about 0.5 to 2.5 turns per inch.

The tensile strength of the finished floss should be between 4 and 25 pounds, although higher tensile strengths are acceptable. The preferred tensile strength is about 6 to 20 pounds. A finished yarn of less than 4 pounds will tend to break easily during use and would be unacceptable for a dental floss, and a finished yarn of more than 25 pounds tensile strength offers no advantages yet is less economical to manufacture. The yarn may be of 200 to 2000 denier, while the preferred dental floss is of 400 to 900 denier for proper hand feel and comfortable use in the mouth.

152

The single end yarn dental floss can be coated with a wax or non-wax coating in accordance with known technology. For example, the yarn may be dipped into a bath of coating material and passed through an oven to evaporate the solvent if a non-wax coating is utilized or through a refrigerated chamber if a waxed coating is utilized in order to solidify same. Another technique which can be utilized comprises pinch rollers delivering a measured layer of coating material as the floss passes over the rollers.

The preferred waxes for coating the dental floss substrate are those that are white or colorless and have a melting point of from 140°F to 210°F. Suitable waxes include beeswax, parafin and microcrystalline waxes, polyethylene glycols such as those sold under the trademark "Carbowax" by Union Carbide Corp., New York, and the like as well as mixtures thereof. The wax comprises about 2% to 40% by weight of the dental floss preferably about 10% to 30% by weight.

For added appeal, the wax portion of the dental floss could carry flavor oils spray dried into suitable watersoluble carriers. These flavors would then be included in the wax during the floss manufacture according to methods known to those familiar with the art. Upon use, the water present in the oral fluids will release the flavor.

If a non-wax coating is desired, a polymeric coating can be utilized. Specific polymeric coatings which are useful in the present invention include:

a.    alkyl monoesters of poly(methylvinylether/maleic acid);

b.  polyvinyl pyrrolidones;

c.  acrylamide/acrylate/butylaminoethyl methacrylate
    polymers.  Polymers of this type are sold by National
    Starch & Chemical Corporation under the trademark
    "Amphomer";

d.  vinyl acetate/crotonic acid/vinyl neodecanoate
    terpolymers.  Terpolymers of this type are sold by
    National Starch & Chemical Corporation under the
    trademark "Resyn 28-2930";

e.  vinyl acetate/crotonic acid copolymers.  Copolymers of
    this type are sold by the National Starch & Chemical
    Corporation under the trademark "Resyn 28-1310";

f.  terpolyamides comprised of the copolymerization
    products of three polyamide precursors, a dicarboxylic
    acid-diamine reaction product, a second but dissimilar
    dicarboxylic acid-diamine reaction product and a
    lactam.  Terpolymers of this type are sold by Belding
    Chemical Industries as the BCI-600 series nylons;

g.  hydroxypropyl cellulose.  Polymers of this type are
    sold by Hercules Incorporated under the trademark
    "Klucel".

The polymeric coating placed on the surface of the yarn
should comprise from about 1 to about 10 percent by weight
of the final product.

As with the wax coating, the non-wax polymeric coatings
can carry flavor oils spray dried into suitable water
soluble carriers.  Furthermore, other ingredients may be
incorporated or impregnated into the wax or non-wax

&J-1152

coating such as fluorides, abrasives and polishing agents, therapeutic agents and the like.

A method of preparing the dental floss of the present invention is as follows. An appropriate yarn is selected, for example DuPont's 840 denier, 140 filament, Type 715 nylon 6,6 yarn or Monsanto's 840 denier, 140 filament, Type A07 nylon 6,6 yarn and a twist of 2 turns per inch is applied. Microcrystalline wax is then heated to a temperature of from about 170°F to 210°F until it is molten and then it is placed in a suitable bath. The twisted yarn is then passed through the wax bath on suitable guides such that when the yarn leaves the molten bath it carries with it an excess of wax. The yarn is drawn through the wax bath at a speed of from about 200 ft/min. to 2,000 ft/min., as desired. The excess wax is then removed from the yarn either by means of wiping or by passing the yarn over sling rollers. The yarn is then passed into a suitable refrigerated chamber having a temperature lower than the setting temperature of the wax thereby setting the wax. The finished product is then wound on large spools to be packaged as desired.

If a non-wax coating is desired, such is dissolved in a suitable solvent and placed in a bath and the above process can be carried out, passing the yarn through an oven suitably heated to evaporate the solvent from the coating. The speed and temperature are chosen to dry the yarn without over heating and thus chemically changing the coating material.

A dental floss as described herein is perceived effective in cleaning between the teeth and exhibits ease of insertion between the teeth as well as ease of handling. The dental floss of this invention is also judged to be of the right flexibility and softness.

In addition to the preferred embodiments described herein, other arrangements and variations within the spirit of the invention and the scope of the appended claims will occur to those skilled in the art.

0172671

CLAIMS

1. A dental floss for cleaning the teeth comprising a single end yarn containing from about 25 to 1,000 filaments.

2. The dental floss of claim 1 containing a wax coating.

3. The dental floss of claim 1 containing a non-wax coating.

4. The dental floss of claim 3 wherein the non-wax coating is selected from the group consisting of alkyl monoesters of poly(methylvinylether/maleic acid), polvinyl pyrrolidones, acrylamide/acrylate/butylaminoethyl methacrylate polymers, vinyl acetate/crotonic acid, vinyl neodecanoate terpolymers, vinyl acetate/crotonic acid copolymers, terpolyamides comprised of the copolymerization products of three polyamide precursors, a dicarboxylic acid-diamine reaction product, a second but dissimilar dicarboxylic acid-diamine reaction product and a lactam, and hydroxypropyl cellulose.

5. The dental floss of any one of claim 1 to 4 wherein the yarn contains from about 55 to 450 filaments.
6. The dental floss of any one of claim 1 to 5 wherein the yarn has a twist of from about 0 to 6 turns per inch.
7. The dental floss of any one of claims 1 to 6 wherein the yarn is of nylon 6, nylon 6,6, rayon, acetate polymer, polyester, polyethylene or polypropylene.
8. The dental floss of claim 7 wherein the yarn is nylon 6,6.